# EUROPEAN PATENT APPLICATION

(11) **EP 1 584 690 A1**
(43) Date of publication of application: **12.10.2005**
(21) Application number: 03777356.1
(22) Date of filing: 08.12.2003
(51) Int. Cl.: C12P 41/00, C12N 9/80

(54) **PROCESS FOR PRODUCING OPTICALLY ACTIVE a-METHYLCYSTEINE DERIVATIVE**

(30) Priority: 10.01.2003 JP 2003005150
(71) Applicant: KANEKA CORPORATION, Osaka-shi, Osaka 530-8288 (JP)
(72) Inventor: UEDA, Makoto, c/o KANEKA CORPORATION, Takasago-shi, Hyogo 676-0027 (JP); NANBA, Hirokazu, c/o KANEKA CORPORATION, Takasago-shi, Hyogo 676-0027 (JP)
(74) Representative: HOFFMANN EITLE
(86) International application number: PCT/JP2003/015682
(87) International publication number: WO 2004/063385

(57) **Abstract**

A process for conveniently and industrially producing an optically active α-methylcysteine derivative, which is useful as an intermediate of medicines and the like, from an inexpensive and readily available material is provided.

The present invention relates to a process for producing a racemic or optically active α-methylcysteine derivative including a step of hydrolyzing a racemic or optically active N-carbamyl-α-methylcysteine derivative by treating with decarbamylase, and a process for producing an optically active α-methylcysteine derivative and an optically active N-carbamyl-α-methylcysteine derivative having a configuration opposite to that of the compound including a step of stereoselectively hydrolyzing a racemic N-carbamyl-α-methylcysteine derivative by treating with decarbamylase.

## Description

### Technical Field

The present invention relates to a process for producing an α-methylcysteine derivative, which is a kind of amino acid having two different substituents at the α position and is useful as an intermediate of medicines and the like. In more detail, the present invention relates to a process for producing an optically active α-methylcysteine derivative and an optically active N-carbamyl-α-methylcysteine derivative, in particular, L-α-methylcysteine derivative, the process including a step of treating a racemic or optically active N-carbamyl-α-methylcysteine derivative with decarbamylase.

### Background Art

The following processes are known as processes for producing optically active α-methylcysteine derivatives.
(1) A process of asymmetrically methylating an optically active thiazolidine compound produced from optically active cysteine and pivalaldehyde (PCT Japanese Translation Patent Publication No. 2000-515166, PCT Publication Nos. WO01/72702 and WO01/72703).
(2) A process of asymmetrically thiomethylating an optically active thiazolidine compound produced from optically active alanine and benzaldehyde (Tetrahedron, 1999, vol. 55, p. 10685).
(3) A process of methylating a thiazoline compound produced from cysteine and cyanobenzene, and separating and purifying the resultant racemic thiazoline compound by chiral HPLC (Synlett, 1994, vol. 9, p. 702).
(4) A process of asymmetrically bromomethylating an optically active diketopiperazine compound synthesized from optically active valine and alanine, and replacing a bromine atom of the resultant compound with an alkali metal alkyl thiolate (J. Org. Chem., 1992, vol. 57, p. 5568).
(5) A process of synthesizing optically active aziridine from optically active 2-methylglycidol produced by Sharpless asymmetric oxidation of 2-methyl-2-propen-1-ol, and reacting the resultant compound with a thiol (J. Org. Chem. , 1995, vol. 60, p. 790).
(6) A process of methylating an aminomalonic acid derivative, asymmetrizing the derivative with pig liver esterase (hereinafter abbreviated as PLE), and reacting the resultant asymmetric ester with an alkali metal salt of thioacetic acid (J. Am. Chem. Soc., 1993, vol. 115, p.8449).

Hitherto, a process for producing a racemic or optically active α-methylcysteine derivative by hydrolyzing an N-carbamyl-α-methylcysteine derivative with an enzyme has not yet been known.

However, processes (1) to (4) require a low temperature reaction using an expensive base such as butyllithium. Process (5) takes many complex steps and requires many expensive reagents. In process (6), although asymmetrization of a diester using PLE as an esterase is considered as a key, the mass production of PLE is difficult. Therefore, it is difficult to secure PLE stably on an industrial scale and this process is not practical. Thus, all the processes have a problem to be solved as an industrial production process for an optically active α-methylcysteine derivative.

On the other hand, as a method for solving the above problem, the present inventors have found a process for producing an α-methyl-L-cysteine derivative (Japanese Patent Application No. 2002-164598). In this process, a racemic N-carbamyl-α-methylcysteine derivative is treated with hydantoinase to selectively cyclize the D-isomer, thereby producing an N-carbamyl-α-methyl-L-cysteine derivative and a D-5-methyl-5-thiomethylhydantoin derivative. Furthermore, the resultant N-carbamyl-α-methyl-L-cysteine derivative is chemically hydrolyzed to produce the α-methyl-L-cysteine derivative. However, this process requires a large amount of acid or alkali in the step of hydrolyzing the N-carbamyl-α-methyl-L-cysteine derivative, and in addition, the reaction time is long. Therefore, there is a room for further improvement in this process.

### Disclosure of Invention

It is an object of the present invention to provide a process for conveniently producing an optically active α-methylcysteine derivative, which is useful as an intermediate of medicines, from an inexpensive and readily available material, the process being practical for industrial production.

As a result of intensive study for solving these problems, the present inventors have found a process for producing a racemic or optically active α-methylcysteine derivative by hydrolyzing a racemic or optically active N-carbamyl-α-methylcysteine derivative with a decarbamylase, and a process for producing an optically active α-methylcysteine derivative and an optically active N-carbamyl-α-methylcysteine derivative by stereoselectively hydrolyzing a racemic N-carbamyl-α-methylcysteine derivative with a decarbamylase. These findings resulted in completion of the present invention.

In other words, the present invention relates to a process for producing a racemic or optically active α-methylcysteine derivative represented by general formula (2) : 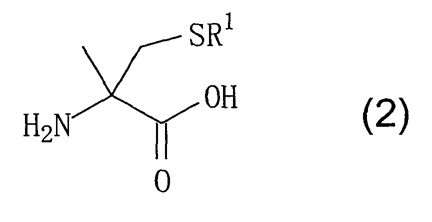 (wherein R¹ represents a substituted or unsubstituted alkyl group having 1 to 20 carbon atoms, a substituted or unsubstituted aralkyl group having 7 to 20 carbon atoms, or a substituted or unsubstituted aryl group having 6 to 20 carbon atoms), the process including hydrolyzing a racemic or optically active N-carbamyl-α-methylcysteine derivative represented by general formula (1): 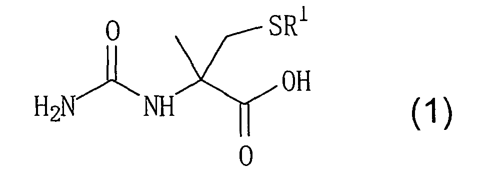 (wherein R¹ is as defined above) by treating with decarbamylase.

Also, the present invention relates to a process for producing an optically active α-methylcysteine derivative represented by formula (2) and an optically active N-carbamyl-α-methylcysteine derivative having a configuration opposite to that of the compound, the process including stereoselectively hydrolyzing a racemic N-carbamyl-α-methylcysteine derivative represented by formula (1) by treating with decarbamylase.

The present invention will now be described in detail.

In an N-carbamyl-α-methylcysteine derivative (1) used in the present invention, R¹ represents a substituted or unsubstituted alkyl group having 1 to 20 carbon atoms, a substituted or unsubstituted aralkyl group having 7 to 20 carbon atoms, or a substituted or unsubstituted aryl group having 6 to 20 carbon atoms.

Examples of the alkyl group having 1 to 20 carbon atoms include linear alkyl groups such as methyl, ethyl, and propyl groups; and branched alkyl groups such as isopropyl, isobutyl, tert-butyl, neopentyl, and tert-pentyl groups. Examples of the aralkyl group having 7 to 20 carbon atoms include benzyl, p-methoxybenzyl, phenethyl, and naphthylmethyl groups. Examples of the aryl group having 6 to 20 carbon atoms include phenyl and naphthyl groups.

The alkyl group, the aralkyl group, and the aryl group may be unsubstituted or substituted. Examples of a substituent include amino, hydroxyl, phenyl, aryl, alkanoyl, alkenyl, alkynyl, and alkoxyl groups, and a halogen atom.

In terms of the ease of deprotection, among these groups, R¹ is preferably a substituted or unsubstituted tertiary alkyl group having 4 to 15 carbon atoms or a substituted or unsubstituted aralkyl group having 7 to 20 carbon atoms. Specifically, R¹ is preferably a tert-butyl, tert-pentyl, tert-hexyl, or benzyl group, and more preferably, a tert-butyl group.

For example, a racemic N-carbamyl-α-methylcysteine derivative represented by formula (1) can be prepared as follows: Thioacetone is converted to hydantoin by a Bucherer method, and the resultant product is hydrolyzed to an α-methylcysteine derivative (Agr. Biol. Chem., 1971, 35, 53-58, Bull. Korean Chem. Soc., 1988, 9, 231-235, and Tetrahedron Asymmetry, 1997, 2913-2920). Subsequently, the resultant product is treated with potassium cyanate.

For example, an optically active N-carbamyl-α-methylcysteine derivative represented by formula (1) can also be prepared as follows: A racemic N-carbamyl-α-methylcysteine derivative is treated with hydantoinase (for example, hydantoinase derived from Pseudomonas putida IFO12996) so as to selectively cyclize the D-isomer. The resultant N-carbamyl-α-methyl-L-cysteine derivative is separated from the D-5-methyl-5-thiomethylhydantoin derivative by, for example, crystallization.

A process for producing a racemic or optically active α-methylcysteine derivative (2) by hydrolyzing a racemic or optically active N-carbamyl-α-methylcysteine derivative (1) with a decarbamylase, and a process for producing an optically active α-methylcysteine derivative (2) by stereoselectively hydrolyzing a racemic N-carbamyl-a-methylcysteine derivative (1) with a decarbamylase will now be described.

Herein, the decarbamylase refers to an enzyme having the activity of producing an α-amino acid derivative by hydrolyzing an N-carbamyl-α-amino acid derivative.

A decarbamylase derived from any origin such as animals, plants, or microorganisms can be used in the present invention so long as the decarbamylase can hydrolyze the N-carbamyl-α-methylcysteine derivative (1). In terms of the ease of availability or preparation, a decarbamylase derived from microorganisms is preferable for industrial use.

Any microorganism can be used as the source of the enzyme so long as the microorganism has the ability of producing the enzyme. Known examples of such a microorganism include microorganisms belonging to genus Achromobacter, Aerobacter, Aeromonas, Agrobacterium, Alcaligenes, Arthrobacter, Bacillus, Blastobacter, Bradyrhizobium, Brevibacterium, genus Comamonas, Flavobacterium, Moraxella, Paracoccus, Pseudomonas, Rhizobium, Serratia, or Sporosarcina, which are disclosed in Japanese Examined Patent Application Publication Nos. 57-18793, 63-20520, and 1-48758, and Japanese Unexamined Patent Application Publication No. 6-233690.

Preferably, an enzyme derived from microorganisms belonging to genus Agrobacterium, Blastobacter, Comamonas, Pseudomonas, or Rhizobium can be used.

More preferably, an enzyme derived from Agrobacterium sp. KNK712 (FERM BP-1900), Rhizobium sp. KNK1415 (FERM BP-4419), or Pseudomonas sp. KNK003A (FERM BP-3181) can be used.

The microorganisms may be a wild strain or a mutant strain having enhanced decarbamylase activity by mutagenization. Furthermore, the microorganisms may be transformed microorganisms prepared using a method such as a gene recombination so as to highly produce the decarbamylase derived from the above microorganisms.

Agrobacterium sp. strain KNK712 (FERM BP-1900), Rhizobium sp. strain KNK1415 (FERM BP-4419), and Pseudomonas sp. strain KNK003A (FERM BP-3181) have been deposited with International Patent Organism Depositary in National Institute of Advanced Industrial Science and Technology since May 31, 1988, September 22, 1993, and December 1, 1990, respectively, under the above accession numbers.

The highly productive transformed microorganisms that efficiently produce decarbamylase can be prepared as described in, for example, PCT Publication No. WO92/10579: A decarbamylase gene is cloned from a microbial strain having decarbamylase activity, a recombinant plasmid with an appropriate vector is then prepared, and an appropriate host microorganism is transformed using the recombinant plasmid. The recombinant DNA technology, which is well known in the related field, is described in, for example, Molecular Cloning 2nd Edition (Cold Spring Harbor Laboratory Press, 1989) and Current Protocols in Molecular Biology (Greene Publishing Associates and Wiley-Interscience).

Examples of such a transformed microorganism that highly produces decarbamylase include Escherichia coli JM109 (pAD108) (FERM BP-3184) including a decarbamylase gene derived from Agrobacterium sp. KNK712 (FERM BP-1900) and Escherichia coli JM109 (pPD304) (FERM BP-3183) including a decarbamylase gene derived from Pseudomonas sp. KNK003A (FERM BP-3181), which are described in PCT publication No. WO92/10579, and Escherichia coli HB101 (pNT4553) (FERM BP-4368) including a decarbamylase gene derived from Agrobacterium sp. KNK712 (FERM BP-1900) having an improved thermal resistance through genetic modification, which is described in PCT publication No. WO94/03613.

Escherichia coli strain JM109 (pAD108) (FERM BP-3184) and Escherichia coli strain JM109 (pPD304) (FERM BP-3183) have been deposited with International Patent Organism Depositary in National Institute of Advanced Industrial Science and Technology since December 1, 1990, under the above accession numbers. Escherichia coli strain HB101 (pNT4553) (FERM BP-4368) has been deposited with International Patent Organism Depositary in National Institute of Advanced Industrial Science and Technology since July 22, 1993, under the above accession number.

The decarbamylase can be produced by culturing the above microorganisms or transformed microorganisms having decarbamylase activity by a normal method. The culture is generally performed with a liquid nutrient medium. Alternatively, the culture can be performed on the surface of a solid medium. The medium generally includes a carbon source and a nitrogen source that can be utilized, and nutrients of inorganic salts essential for the growth of various microorganisms. Furthermore, small amounts of amino acids such as p-hydroxyphenylglycine and phenylglycine; N-carbamyl-α-amino acids such as N-carbamyl-methionine and N-carbamyl-phenylalanine; 5-substituted hydantoins such as 5-p-hydroxyphenylhydantoin and 5-phenylhydantoin; pyrimidine metabolites such as uracil, dihydrouracil, and β-ureide propionic acid; urea; and metal ions such as Fe²⁺, Fe³⁺, Be²⁺, Co²⁺, Al³⁺, Li⁺, Mn²⁺, Mg²⁺, and Cs⁺ are preferably added so as to enhance the production of decarbamylase. The concentration of these substances for enhancing the production of decarbamylase in the medium is selected from the ranges of 0.1 to 10 mM for the metal ions and 0.01 to 1% by weight for the other substances.

The incubation is generally performed at a temperature of 20°C to 85°C, preferably, 25°C to 60°C, and at a pH of 4 to 11, preferably, 5 to 9. Stirring for aeration can facilitate the growth of microorganisms.

In the present invention, a decarbamylase produced by the above microorganisms can be used as the enzyme itself. Alternatively, the decarbamylase can be used as a microorganism having the activity of the enzyme or a processed product of the microorganism. Herein, the processed product of the microorganism includes, for example, a crude extract, organisms prepared by freeze-drying incubated microbial cells, organisms dried with acetone, and a disrupted product of these microbial cells.

Furthermore, the enzyme, the microorganism, or the processed product thereof can be used as an immobilized enzyme prepared by immobilizing the enzyme itself or the microbial cells by a known method. The immobilization can be performed by a method that is well known to those skilled in the art, for example, crosslinking, covalent binding, physical adsorption, or entrapment method. The stabilization of the enzyme by immobilization allows the enzymatic reaction to be performed in a severer temperature range. Thus, the reaction can proceed more efficiently. Furthermore, an advantage such as the decrease in production cost due to the repetitive use of the enzyme and the simplification of the production process can also be expected.

The enzymatic reaction of the present invention can be performed by the following process. The reaction is performed in an aqueous medium using a racemic or optically active N-carbamyl-α-methylcysteine derivative represented by formula (1) as a substrate under the presence of the above decarbamylase. The reaction is performed in a dissolved or suspended state with a charge ratio of the substrate of 0.1% (w/v) to 90% (w/v), preferably, 1% (w/v) to 60% (w/v). The reaction mixture is left to stand or stirred for a while, while the reaction temperature is adequately controlled to be 10°C to 80°C, preferably, 20°C to 60°C, and the pH is maintained at 4 to 9, preferably, 5 to 8. The substrate can be added continuously. The reaction can be performed by a batch process or a continuous process. Also, the reaction of the present invention can be performed using an immobilized enzyme, a membrane reactor, or the like.

Examples of the aqueous medium include appropriate solvents such as water; a buffer; an aqueous medium containing these and a water-soluble organic solvent such as ethanol; and a two-layer system including an aqueous medium and an organic solvent that is difficult to dissolve in water, e.g., ethyl acetate, butyl acetate, toluene, chloroform, or n-hexane. In addition, an anti-oxidant, a surfactant, a coenzyme, and a metal can be added according to need.

Thus, the racemic or optically active N-carbamyl-α-methylcysteine derivative (1) is hydrolyzed to convert to a racemic or optically active α-methylcysteine derivative (2). When the enzymatic reaction proceeds stereoselectively, an optically active α-methylcysteine derivative (2) and an optically active N-carbamyl-α-methylcysteine derivative (1) having a configuration opposite to that of the compound are produced from the racemic N-carbamyl-α-methylcysteine derivative (1).

In terms of the availability of the enzyme and reaction efficiency, preferably, the L-compound (1) is hydrolyzed or the racemic compound (1) is treated so as to hydrolyze the L-isomer selectively, thereby producing the L-isomer of α-methylcysteine derivative (2). In terms of the ease of the substrate preparation, more preferably, the racemic compound (1) is treated so as to hydrolyze the L-isomer selectively.

When a stereoselective hydrolysis is performed, the enzyme derived from Agrobacterium sp. KNK712 (FERM BP-1900), Rhizobium sp. KNK1415 (FERM BP-4419), or Pseudomonas sp. KNK003A (FERM BP-3181) is preferable as the L-isomer-selective decarbamylase, and these microorganisms or a processed product thereof can be preferably used. In addition, as described above, transformed microorganisms such as Escherichia coli JM109 (pAD108) (FERM BP-3184), Escherichia coli JM109 (pPD304) (FERM BP-3183), and Escherichia coli HB101 (pNT4553) (FERM BP-4368), which are modified so as to produce the decarbamylase derived from the above microorganisms, or a processed product of the transformed microorganisms can also be used preferably. The bacterial cells of Escherichia coli HB101 (pNT4553) (FERM BP-4368) or a processed product thereof is more preferable.

The resultant racemic or optically active α-methylcysteine derivative (2) and the racemic or optically active N-carbamyl-α-methylcysteine derivative (1) can be separated and purified by a normal separation method such as extraction, concentration, crystallization, or column chromatography or by a combination of these. The separation and purification can be more readily performed by the following process.

The solubility of the racemic or optically active α-methylcysteine derivative (2) in water is very low at about the neutral pH range, whereas the racemic or optically active N-carbamyl-α-methylcysteine derivative (1) has a relatively high solubility in water. Accordingly, the pH of the reaction mixture is adjusted to be about neutral after the completion of the enzymatic reaction. As a result, the racemic or optically active α-methylcysteine derivative (2) is crystallized and is readily separated from the racemic or optically active N-carbamyl-α-methylcysteine derivative (1) by filtration. In this case, the pH is preferably 4 to 10, more preferably, 6 to 8.

Regarding the resultant filtrate containing the racemic or optically active N-carbamyl-a-methylcysteine derivative (1), the racemic or optically active N-carbamyl-α-methylcysteine derivative (1) is crystallized by acidifying the pH of the filtrate and the crystals can be readily separated by filtration. Although an acid used for this is not particularly limited, examples of the acid include acetic acid, nitric acid, sulfuric acid, and hydrochloric acid. In terms of the cost and easy handling, hydrochloric acid is preferably used. The pH is preferably in the range of 1 to 5, in particular, 2 to 4 because an excessively low pH produces hydantoin by chemical cyclization.

Furthermore, the carbamyl group of the resultant optically active N-carbamyl-α-methylcysteine derivative (1) is removed chemically or using an enzyme having decarbamyl activity, whereby an optically active α-methylcysteine derivative (2) having a configuration opposite to that of the above α-methylcysteine derivative can be readily derived.

### Best Mode for Carrying Out the Invention

The present invention will now be described more specifically with reference to Examples. However, the present invention is not limited to these Examples.

### (REFERENCE EXAMPLE 1) Process for producing racemic 5-methyl-5-tert-butylthiomethylhydantoin

In a reactor with a nitrogen balloon, a 5 wt% aqueous solution of sodium hydroxide (9.6 g) and tert-butylmercaptan (1.13 mL) were mixed at 0°C, and the mixture was stirred for 10 minutes. Subsequently, chloroacetone (0.79mL) was added. The temperature was increased to room temperature and the reaction was performed for 2 hours. This reaction mixture was pale yellow and was separated to two phases. A Dimroth condenser was provided to the reactor and sodium cyanide (588 mg), ammonium hydrogencarbonate (2.77 g), and 30 wt% aqueous ammonia (3.1 mL) were added to provide a homogeneous solution. Subsequently, the temperature was increased to 55°C to 60°C. The reaction mixture was heated and stirred for 6 hour and was then cooled to 0°C. Concentrated hydrochloric acid was added to the reaction mixture so that the pH was adjusted to 7.0 to 7.6. The resultant white crystals (1.84 g) were filtered (yield 84.8%). The crystals were confirmed to be the target compound by ¹H-NMR analysis.

### (REFERENCE EXAMPLE 2) Process for producing racemic N-carbamyl-S-tert-butyl-α-methylcysteine

Racemic 5-methyl-5-thiomethylhydantoin (4.77 g) was dissolved in a 10 wt% aqueous solution of sodium hydroxide (75 g) and the mixture was refluxed for 72 hours. The reaction mixture was left to cool to room temperature and a part of the reaction mixture was then sampled. The formation of racemic S-tert-butyl-α-methylcysteine was confirmed by an analysis using high performance liquid chromatography (HPLC). The pH was adjusted to 8 with concentrated hydrochloric acid and the mixture was then heated to 70°C. A solution dissolved potassium cyanate (2.07 g) in distilled water (10 mL) was added dropwise over a period of 20 minutes. After the dropwise addition, the reaction mixture was stirred for 5 hours. Subsequently, a part of the reaction mixture was sampled and analyzed by HPLC. Since an unreacted amino acid was detected, a solution dissolved potassium cyanate (4.14 g) in distilled water (20 mL) was further added dropwise over a period of 20 minutes. After the dropwise addition, the reaction mixture was further stirred for 1 hour. The reaction mixture was left to cool to room temperature, and the pH was adjusted to 2 with concentrated hydrochloric acid. The precipitated solid was recovered by filtering. The resultant solid was washed with water and dried. Subsequently, the solid was confirmed to be the target compound by ¹H-NMR analysis (3.38 g, yield 66%).
Analytical conditions for HPLC;
Column: COSMOSIL 5C18-ARII (4. 6 mm in diameter × 250 mm, from Nacalai Tesque, Inc.), Mobile phase: 10 mM phosphoric
acid-potassium dihydrogenphosphate buffer (pH 2.0)/acetonitrile = 97/3, Column temperature: 40°C,
Measurement wavelength: 210 nm, Flow rate: 1.0 mL/min.

### (EXAMPLE 1) Production of S-tert-butyl-α-methyl-L-cysteine using bacteria of genus Agrobacterium

Agrobacterium sp. KNK712 (FERM BP-1900) was inoculated in a culture medium A (10 mL) (polypeptone 10 g, a meat extract 10 g, yeast extract 5 g, glycerin 5 g, potassium dihydrogenphosphate 5 g, disodium hydrogenphosphate 5 g, and water 1 L, pH 6.5 before sterilization) that was sterilized in a test tube, and the solution was incubated with shaking at 30°C for 24 hours. The culture broth (1 mL) was inoculated in a culture medium B (100 mL) (glycerin 25 g, sucrose 5 g, yeast extract 4 g, potassium dihydrogenphosphate 5 g, disodium hydrogenphosphate 5 g, magnesium phosphate heptahydrate 1 g, manganese chloride tetrahydrate 0.01 g, and water 1 L, pH 6.5 before sterilization) that was sterilized in a Sakaguchi flask. Urea (0.2 g) and N-carbamyl-D-p-hydroxyphenylglycine (0.2 g) that were sterilized by filtration were further added to the solution. The solution was incubated with shaking at 33°C for 36 hours. Bacterial cells harvested by centrifuging the resultant culture broth (16.5 mL) were suspended in a 0.2 M N-2-hydroxyethylpiperazine-N'-2-ethanesulfonic acid (HEPES)/sodium hydroxide buffer (pH 6.5, 1.5 mL). Racemic N-carbamyl-S-tert-butyl-α-methylcysteine (75 mg) and a 75% aqueous solution of cysteamine (0.2 µL) were added to the suspension. Furthermore, the pH was adjusted to 6.5 with a 10 N aqueous solution of sodium hydroxide. The suspension was stirred in a nitrogen atmosphere at 40°C for 25 hours. During the stirring, the pH was kept at about 6.5 by adding 6 N hydrochloric acid. After the reaction was completed, the product was analyzed by HPLC. According to the results, S-tert-butyl-α-methylcysteine was produced in a conversion ratio of 10.0% and an optical purity of 33.7%ee. The resultant S-tert-butyl-α-methylcysteine was confirmed to be the L-isomer by comparing the retention time by HPLC analysis of the product with that of a specimen synthesized separately.
Analytical conditions for HPLC (measurement of conversion ratio);
Column: COSMOSIL 5C18-ARII (4.6 mm in diameter × 250 mm, from Nacalai Tesque, Inc.), Mobile phase: 10 mM phosphoric
acid/potassium dihydrogenphosphate buffer (pH 2.0)/acetonitrile = 8/2, Column temperature: 40°C,
Measurement wavelength: 210 nm, Flow rate: 1.0 mL/min.
Analytical conditions for HPLC (measurement of optical purity of S-tert-butyl-α-methylcysteine);
Column: SUMICHIRAL OA-5000 (4.6 mm in diameter × 150 mm, from Sumika Chemical Analysis Service, Ltd.), Mobile phase: 2 mM aqueous solution of copper sulfate/acetonitrile = 85/15,
Column temperature: 23°C, Measurement wavelength: 254 nm, Flow rate: 1.5 mL/min.

### (EXAMPLE 2) Production of S-tert-butyl-α-methyl-L-cysteine using bacteria of genus Rhizobium

Bacterial cells harvested by centrifuging a culture broth (36 mL) of Rhizobium sp. KNK1415 (FERM BP-4419) incubated by the same method as that in Example 1 were suspended in a 0.2 M HEPES/sodium hydroxide buffer (pH 6.5, 1.5 mL). Racemic N-carbamyl-S-tert-butyl-α-methylcysteine (75 mg) and a 75% aqueous solution of cysteamine (0.2 µL) were added to the suspension. Furthermore, the pH was adjusted to 6.5 with a 10 N aqueous solution of sodium hydroxide. The suspension was stirred in a nitrogen atmosphere at 40°C for 25 hours. During the stirring, the pH was kept at about 6.5 by adding 6 N hydrochloric acid. After the reaction was completed, the product was analyzed as in Example 1. According to the results, S-tert-butyl-α-methyl-L-cysteine was produced in a conversion ratio of 9.5% and an optical purity of 2.4%ee.

### (EXAMPLE 3) Production of S-tert-butyl-α-methyl-L-cysteine using bacteria of genus Pseudomonas

Pseudomonas sp. KNK003A (FERM BP-3181) was inoculated in a culture medium C (10 mL) (tryptone 16 g, yeast extract 10 g, sodium chloride 5 g, and water 1 L, pH 7 before sterilization) that was sterilized in a test tube, and the solution was incubated with shaking at 45°C for 27 hours. The culture solution (1 mL) was inoculated in a culture medium D (100 mL) (glycerin 10 g, yeast extract 0.03 g, potassium dihydrogenphosphate 3.5 g, disodium hydrogenphosphate 3.5 g, magnesium phosphate heptahydrate 0.5 g, manganese chloride tetrahydrate 0.02 g, ferrous sulfate heptahydrate 0.01 g, calcium carbonate 1 g, and water 1 L, pH 7 before sterilization) that was sterilized in a Sakaguchi flask. Glucose (0.5 g) that was separately sterilized and N-carbamyl-D-alanine (0.2 g) that was separately sterilized by filtration were further added to the solution. The solution was incubated with shaking at 45°C for 61 hours. Bacterial cells harvested by centrifuging the resultant culture broth (30 mL) were suspended in a 0.2 M HEPES/sodium hydroxide buffer (pH 6.5, 1.5 mL). Racemic N-carbamyl-S-tert-butyl-α-methylcysteine (75 mg) and a 75% aqueous solution of cysteamine (0.2 µL) were added to the suspension. Furthermore, the pH was adjusted to 6.5 with a 10 N aqueous solution of sodium hydroxide. The suspension was stirred in a nitrogen atmosphere at 40°C for 20 hours. During the stirring, the pH was kept at about 6.5 by adding 6 N hydrochloric acid. After the reaction was completed, the product was analyzed as in Example 1. According to the results, S-tert-butyl-α-methyl-L-cysteine was produced in a conversion ratio of 4.2% and an optical purity of 11.7%ee.

### (EXAMPLE 4) Production of S-tert-butyl-α-methyl-L-cysteine using transformed Escherichia coli HB101 (pNT4553)

Escherichia coli HB101 (pNT4553) (FERM BP-4368) including a decarbamylase gene derived from Agrobacterium sp. KNK712 (FERM BP-1900) having an improved thermal resistance through genetic modification was inoculated in the culture medium C (10 mL) (tryptone 16 g, yeast extract 10 g, sodium chloride 5 g, and water 1 L, pH 7 before sterilization) that was sterilized in a test tube. Ampicillin sodium (1 mg) that was separately sterilized by filtration was further added to the solution, and the solution was incubated with shaking at 37°C for 12 hours. The culture broth (3.5 mL) was inoculated in the culture medium C (350 mL) that was sterilized in a Sakaguchi flask. Ampicillin sodium (35 mg) that was separately sterilized by filtration was further added to the solution. The solution was incubated with shaking at 37°C for 30 hours. Bacterial cells harvested by centrifuging the resultant culture broth (3.6 mL) were suspended in a 0.2 M HEPES/sodium hydroxide buffer (pH 6.5, 1.5 mL). Racemic N-carbamyl-S-tert-butyl-α-methylcysteine (75 mg) and a 75% aqueous solution of cysteamine (0.2 µL) were added to the suspension. Furthermore, the pH was adjusted to 6.5 with a 10 N aqueous solution of sodium hydroxide. The suspension was stirred in a nitrogen atmosphere at 40°C for 20 hours. During the stirring, the pH was kept at about 6.5 by adding 6 N hydrochloric acid. After the reaction was completed, the product was analyzed by HPLC. According to the results, S-tert-butyl-α-methyl-L-cysteine was produced in a conversion ratio of 4.2% and an optical purity of 11.7%ee. Also, N-carbamyl-S-tert-butyl-α-methylcysteine was produced in a conversion ratio of 61.8% and an optical purity of 6.1%ee. The resultant N-carbamyl-S-tert-butyl-α-methylcysteine was confirmed to be the D-isomer by comparing the retention time by HPLC analysis of the product with that of a specimen synthesized separately.
Analytical conditions for HPLC (measurement of optical purity of N-carbamyl-S-tert-butyl-α-methylcysteine);
Column: CHIRALPAK AS (4.6 mm in diameter × 250 mm, from Daicel Chemical Industries, Ltd.), Mobile phase:
hexane/isopropanol/trifluoroacetic acid = 85/15/0.1, Column temperature: 30°C, Measurement wavelength: 210 nm, Flow rate: 0.5 mL/min.

### (EXAMPLE 5) Production of S-tert-butyl-α-methyl-L-cysteine using immobilized decarbamylase

Escherichia coli HB101 (pNT4553) (FERM BP-4368) was cultured according to the culture method described in PCT publication No. WO94/03613, and the bacterial cells were harvested. Subsequently, an enzyme solution was prepared by disruption with ultrasonic waves, according to the process for preparing an immobilized enzyme described in PCT publication No. WO92/22643. An anion-exchange resin Duolite A-568, which was an immobilization support, was added to the enzyme solution to adsorb the enzyme. Furthermore, a crosslinking process was performed with glutaraldehyde to prepare an immobilized decarbamylase. Subsequently, a 0.2 M HEPES/sodium hydroxide buffer (pH 6.5, 1.5 mL) and a 75% aqueous solution of cysteamine (0.2 µL) were added to racemic N-carbamyl-S-tert-butyl-α-methylcysteine (75 mg). Furthermore, the pH was adjusted to 6.5 with a 10 N aqueous solution of sodium hydroxide. The above immobilized decarbamylase (75 mg by wet weight) was added to the solution, and the solution was stirred in a nitrogen atmosphere at 40°C for 27 hours. During the stirring, the pH was kept at about 6.5 by adding 6 N hydrochloric acid. After the reaction was completed, the product was analyzed by the same methods as those in Examples 1 and 4. According to the results, S-tert-butyl-α-methyl-L-cysteine was produced in a conversion ratio of 64.6% and an optical purity of 29.1%ee. Also, N-carbamyl-S-tert-butyl-α-methyl-D-cysteine was produced in a conversion ratio of 35.4% and an optical purity of 21.1%ee.

### (EXAMPLE 6) Production of S-tert-butyl-α-methyl-L-cysteine using transformed Escherichia coli HB101 (pNT4553)

Bacterial cells harvested by centrifuging a culture broth (60 mL) of Escherichia coli HB101 (pNT4553) (FERM BP-4368) cultured by the same method as that in Example 4 were suspended in a 1 mM aqueous solution of cysteamine (10 mL). N-Carbamyl-S-tert-butyl-α-methyl-L-cysteine (optical purity 99.6%ee, 500 mg) was added to the suspension. Furthermore, the pH was adjusted to 6.5 with a 10 N aqueous solution of sodium hydroxide. The suspension was stirred in a nitrogen atmosphere at 40°C for 25 hours. During the stirring, the pH was kept at about 6.5 by adding 2 wt o sulfuric acid. After the reaction was completed, the product was analyzed by the same method as that in Example 4. According to the result, S-tert-butyl-α-methyl-L-cysteine was produced in a conversion ratio of 98.7%.

### Industrial Applicability

As described above, according to the present invention, an optically active α-methylcysteine derivative, which is useful as an intermediate of medicines and the like, can be produced from an inexpensive and readily available material by a process that is convenient and industrially practicable.

### Depositary institution of organism specimens

The following notation relates to microorganism or organism specimens described in detailed description of the invention.

Name of depositary institution: International Patent Organism Depositary (IPOD), National Institute of Advanced Industrial Science and Technology
Address: Central 6, 1-1-1, Higashi, Tsukuba-shi, Ibaraki-ken, 305-8566 Japan
(1) Accession number: IPOD FERM BP-1900
   Date of depositing: May 31, 1988
(2) Accession number: IPOD FERM BP-4419
   Date of depositing: September 22, 1993
(3) Accession number: IPOD FERM BP-3181
   Date of depositing: December 1, 1990
(4) Accession number: IPOD FERM BP-4368
   Date of depositing: July 22, 1993
(5) Accession number: IPOD FERM BP-3184
   Date of depositing: December 1, 1990
(6) Accession number: IPOD FERM BP-3183
   Date of depositing: December 1, 1990

## Claims

1. A process for producing a racemic or optically active α-methylcysteine derivative represented by general formula (2) : 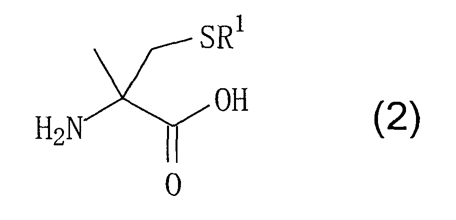 (wherein R¹ represents a substituted or unsubstituted alkyl group having 1 to 20 carbon atoms, a substituted or unsubstituted aralkyl group having 7 to 20 carbon atoms, or a substituted or unsubstituted aryl group having 6 to 20 carbon atoms), the process comprising a step of hydrolyzing a racemic or optically active N-carbamyl-α-methylcysteine derivative represented by general formula (1): 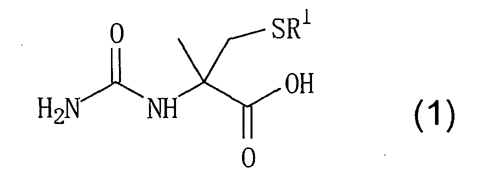 (wherein R¹ is as defined above) by treating with decarbamylase.

2. The process according to claim 1, wherein the N-carbamyl-α-methylcysteine derivative (1) and the resultant α-methylcysteine derivative (2) are optically active.

3. The process according to claim 1 or 2, wherein the N-carbamyl-α-methylcysteine derivative (1) and the resultant α-methylcysteine derivative (2) are L-isomers.

4. A process for producing an optically active α-methylcysteine derivative represented by general formula (2): 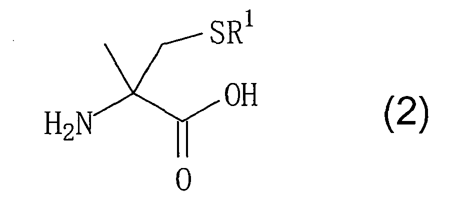 (wherein R¹ represents a substituted or unsubstituted alkyl group having 1 to 20 carbon atoms, a substituted or unsubstituted aralkyl group having 7 to 20 carbon atoms, or a substituted or unsubstituted aryl group having 6 to 20 carbon atoms) and an optically active N-carbamyl-α-methylcysteine derivative having a configuration opposite to that of the compound, the process comprising a step of stereoselectively hydrolyzing a racemic N-carbamyl-α-methylcysteine derivative represented by general formula (1): 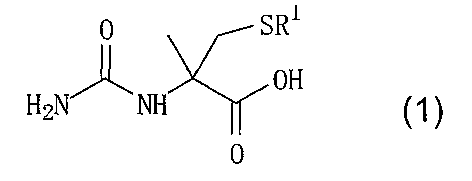 (wherein R¹ is as defined above) by treating with decarbamylase.

5. The process according to claim 4, wherein the resultant α-methylcysteine derivative (2) is an L-isomer.

6. The process according to any one of claims 1 to 5, wherein the decarbamylase is derived from microorganisms belonging to genus Agrobacterium, Rhizobium, or Pseudomonas.

7. The process according to any one of claims 1 to 5, wherein the decarbamylase is derived from Agrobacterium sp. KNK712 (FERM BP-1900), Rhizobium sp. KNK1415 (FERM BP-4419), or Pseudomonas sp. KNK003A (FERM BP-3181).

8. The process according to any one of claims 1 to 5, wherein the decarbamylase is derived from Escherichia coli HB101 (pNT4553) (FERM BP-4368).

9. The process according to any one of claims 1 to 8, wherein the decarbamylase is used in the form of an immobilized enzyme.

10. The process according to any one of claims 1 to 9, wherein R¹ is a substituted or unsubstituted tertiary alkyl group having 4 to 15 carbon atoms.

11. The process according to any one of claims 1 to 9, wherein R¹ is a tert-butyl group.
